# EUROPEAN PATENT APPLICATION

(11) **EP 3 982 122 A1**
(43) Date of publication of application: **13.04.2022**
(21) Application number: 20817957.2
(22) Date of filing: 03.06.2020
(51) Int. Cl.: G01N 33/542

(54) **HOMOGENEOUS IMMUNOASSAY METHOD USING PEPTIDE HAVING MULTIPLE FLUOROCHROMES JOINED THERETO**

(30) Priority: 05.06.2019 JP 2019105006
(71) Applicant: Tokyo Institute of Technology, Tokyo 152-8550 (JP)
(72) Inventor: UEDA, Hiroshi, Tokyo 152-8550 (JP); KITAGUCHI, Tetsuya, Tokyo 152-8550 (JP); OHMURO, Yuki, Tokyo 152-8550 (JP); YASUDA, Takanobu, Tokyo 152-8550 (JP); INOUE, Akihito, Tokyo 152-8550 (JP)
(74) Representative: J A Kemp LLP
(86) International application number: PCT/JP2020/021899
(87) International publication number: WO 2020/246495

(57) **Abstract**

As a simple antigen detection approach using Q-body, provided is a kit for antigen detection, comprising: a first peptide conjugated with two fluorescent dyes capable of forming a FRET pair or a fluorescent dye and a luminescent material capable of forming a BRET pair, and an antibody fragment with a second peptide added thereto, wherein 1) one of the first peptide and the second peptide is positively charged, and the other peptide is negatively charged, and 2) the first peptide and the second peptide are capable of forming a coiled coil.

## Description

### [Technical Field]

The present invention relates to a kit for antigen detection and an antigen detection method using a peptide conjugated with a plurality of fluorescent dyes.

### [Background Art]

Immunoassay is currently an increasing important assay technique in clinical diagnosis. Not only improvement in sensitivity and specificity but also the rapidness and convenience of assay are major factors for adopting each individual immunoassay. In current mainstream immunoassay, a sandwich method for protein biomarker detection and a competitive method for low-molecule detection are used as assay principles. However, both of these methods are often enzyme immunoassay which involves reaction and washing several times followed by the measurement of enzymatic activity by typically using a label, and thus require labor and a time of several hours for assay. In contrast to this, homogeneous immunoassay has been developed in which a sample and an assay reagent are mixed and reacted for detection.

The present inventor has successfully constructed in recent years an antibody Quenchbody (Q-body), which emits light upon binding to an antigen, as a rapid and highly sensitive assay element that may be used in such homogeneous immunoassay, and filed patent applications and published a paper, etc. (Patent Literature 1, Patent Literature 2, and Non Patent Literature 1). Q-body is a fluorescently labeled antibody in which one or two particular locations near an antigen binding site of the antibody are labeled with a fluorescent dye such as TAMRA via a short linker. The dye interacts with an amino acid (typically tryptophan) in the antibody to cause a quenched state, whereas the addition of the antigen cancels the quenching, leading to light emission.

### [Citation List]

### [Patent Literature]

[Patent Literature 1] International Publication No. WO 2011/061944
[Patent Literature 2] International Publication No. WO 2013/065314

### [Non Patent Literature]

[Non Patent Literature 1] R. Abe et al., J. Am. Chem. Soc., 2011, 133 (43), 17386-17394)

### [Summary of Invention]

### [Technical Field]

For exploiting Q-body for a wide range of purposes, it is desirable that antigens be able to be more simply detected. The present invention has been made under such a background, and an object of the present invention is to provide an approach that can simply detect an antigen using Q-body.

### [Solution to Problem]

The present inventor has conducted diligent studies to attain the object and consequently completed the present invention by finding that when Fab harboring E peptide (Litowski et al., J. Biol. Chem. 277, 37272-9 (2002); and Yoshiaki Yano, Seibutsu Butsuri (journal of the Biophysical Society of Japan), 54, 323-324, 2014) is mixed with K peptide (Litowski et al., J. Biol. Chem. 277, 37272-9 (2002); and Yoshiaki Yano, Seibutsu Butsuri (journal of the Biophysical Society of Japan), 54, 323-324, 2014) labeled with TAMRA and FITC, FRET Coiled Q-body (FRET CQ-body) can be prepared which enables an antigen to be detected from change in color.

Specifically, the present invention provides the following [1] to [7].
[1] A kit for antigen detection, comprising: a first peptide conjugated with two fluorescent dyes capable of forming a FRET pair or a fluorescent dye and a luminescent material capable of forming a BRET pair, and an antibody fragment with a second peptide added thereto, wherein 1) one of the first peptide and the second peptide is positively charged, and the other peptide is negatively charged, and 2) the first peptide and the second peptide are capable of forming a coiled coil.
[2] The kit for antigen detection according to [1], wherein the first peptide comprises repeats of an amino acid sequence: X1-X2-X3-X4-X5-X6-X7 (wherein X1 and X6 each represent a positively charged amino acid, X7 represents a negatively charged amino acid, X2 and X5 each represent a hydrophobic amino acid, and X3 and X4 each represent any amino acid), and the second peptide comprises repeats of an amino acid sequence: X8-X9-X10-X11-X12-X13-X14 (wherein X8 and X13 each represent a negatively charged amino acid, X14 represents a positively charged amino acid, X9 and X12 each represent a hydrophobic amino acid, and X10 and X11 each represent any amino acid).
[3] The kit for antigen detection according to [1], wherein the first peptide comprises repeats of an amino acid sequence: Lys Ile Ala Ala Leu Lys Glu, and the second peptide comprises repeats of an amino acid sequence: Glu Ile Ala Ala Leu Glu Lys.
[4] The kit for antigen detection according to [1], wherein the first peptide consists of the amino acid sequence as set forth in SEQ ID NO: 1, and the second peptide consists of the amino acid sequence as set forth in SEQ ID NO: 2.
[5] The kit for antigen detection according to any of [1] to [4], wherein the two fluorescent dyes capable of forming a FRET pair are a rhodamine-based fluorescent dye and a fluorescein-based fluorescent dye.
[6] The kit for antigen detection according to any of [1] to [5], wherein the antibody fragment is Fab or camelid heavy chain antibody-derived VHH.
[7] A method for detecting an antigen in a sample, comprising the following steps (1) and (2):
   (1) contacting the sample with the kit according to any of [1] to [6]; and
   (2) detecting the fluorescence of the fluorescent dye(s) conjugated with the first peptide.

The present specification encompasses the contents described in the specification and/or drawings of Japanese Patent Application No. 2019-105006, on which the priority of the present application is based.

### [Advantageous Effects of Invention]

The present invention provides a novel kit for antigen detection and antigen detection method. The kit and the method can be used in, for example, the field of sample analysis or drug testing, the field of portable sample analysis kits, and the field of clinical diagnosis.

### [Brief Description of Drawings]

[Figure 1] Figure 1 shows a fluorescence spectrum (anti-BGP E4-Fab/K2-FITC-K2C-TMR) of FRET CQ-body before and after antigen addition. The broken line depicts the fluorescence spectrum of FRET CQ-body before antigen addition. The solid line depicts the fluorescence spectrum of FRET CQ-body after antigen addition. By the antigen addition, the fluorescence intensity of FITC became 0.47-fold, and the fluorescence intensity of TMR became 1.74-fold.
[Figure 2] Figure 2 shows a fluorescence spectrum (anti-BGP E4-Fab/K2-FITC-K2C-TMR) after normalization.
[Figure 3] Figure 3 shows a net fluorescence response spectrum (anti-BGP E4-Fab/K2-FITC-K2C-TMR) in antigen addition. The broken line depicts the fluorescence spectrum of FRET CQ-body before antigen addition. The solid line depicts the fluorescence spectrum of FRET CQ-body after antigen addition. By the antigen addition, the fluorescence intensity of TMR became 4.34-fold.
[Figure 4] Figure 4 shows a fluorescence spectrum (anti-MTX E4-VHH/K2-FITC-K2C-TMR) of FRET CQ-body before and after antigen addition. The broken line depicts the fluorescence spectrum of FRET CQ-body before antigen addition. The solid line depicts the fluorescence spectrum of FRET CQ-body after antigen addition.
[Figure 5] Figure 5 shows a fluorescence spectrum (anti-MTX E4-VHH/K2-FITC-K2C-TMR) after normalization.
[Figure 6] Figure 6 shows a net fluorescence response spectrum (anti-MTX E4-VHH/K2-FITC-K2C-TMR) in antigen addition. The broken line depicts the fluorescence spectrum of FRET CQ-body before antigen addition. The solid line depicts the fluorescence spectrum of FRET CQ-body after antigen addition.
[Figure 7] Figure 7 shows a fluorescence spectrum (anti-BGP E4-Fab/FITC-K4C-TMR) of FRET CQ-body before and after antigen addition. The broken line depicts the fluorescence spectrum of FRET CQ-body before antigen addition. The solid line depicts the fluorescence spectrum of FRET CQ-body after antigen addition.
[Figure 8] Figure 8 shows a fluorescence spectrum (anti-BGP E4-Fab/FITC-K4C-TMR) after normalization.
[Figure 9] Figure 9 shows a net fluorescence response spectrum (anti-BGP E4-Fab/FITC-K4C-TMR) in antigen addition. The broken line depicts the fluorescence spectrum of FRET CQ-body before antigen addition. The solid line depicts the fluorescence spectrum of FRET CQ-body after antigen addition.
[Figure 10] Figure 10 shows a fluorescence spectrum (anti-MTX E4-VHH/FITC-K4C-TMR) of FRET CQ-body before and after antigen addition. The broken line depicts the fluorescence spectrum of FRET CQ-body before antigen addition. The solid line depicts the fluorescence spectrum of FRET CQ-body after antigen addition.
[Figure 11] Figure 11 shows a fluorescence spectrum (anti-MTX E4-VHH/FITC-K4C-TMR) after normalization.
[Figure 12] Figure 12 shows a net fluorescence response spectrum (anti-MTX E4-VHH/FITC-K4C-TMR) in antigen addition. The broken line depicts the fluorescence spectrum of FRET CQ-body before antigen addition. The solid line depicts the fluorescence spectrum of FRET CQ-body after antigen addition.
[Figure 13] Figure 13 is a diagram showing the partial structure of pYD1(E4-VHH/MTX) (Figure 13, upper) and the partial structure of pYD1(E4) (Figure 13, lower).
[Figure 14-1] Figure 14-1 shows a FACS profile of an E4-displaying yeast labeled with FITC-K4-TAMRA.
[Figure 14-2] Figure 14-2 shows a FACS profile of an E4-displaying yeast labeled with K2-FITC-K2-TAMRA.
[Figure 15-1] Figure 15-1 shows a FACS profile (antigen MTX absent) of an E4-VHH/MTX-displaying yeast labeled with FITC-K4-TAMRA.
[Figure 15-2] Figure 15-2 shows a FACS profile (antigen MTX present) of an E4-VHH/MTX-displaying yeast labeled with FITC-K4-TAMRA.
[Figure 16-1] Figure 16-1 shows a FACS profile (antigen MTX absent) of an E4-VHH/MTX-displaying yeast labeled with K2-FITC-K2-TAMRA.
[Figure 16-2] Figure 16-2 shows a FACS profile (antigen MTX present) of an E4-VHH/MTX-displaying yeast labeled with K2-FITC-K2-TAMRA.
[Figure 17] Figure 17 shows a fluorescence spectrum (anti-BGP E4-Fab/FITC-K4C-TMR) of FRET CQ-body at varying antigen concentrations.
[Figure 18] Figure 18 is a graph (anti-BGP E4-Fab/FITC-K4C-TMR) showing the relationship between an antigen concentration and a normalized fluorescence peak ratio (fluorescence peak of TAMRA/fluorescence peak of fluorescein).
[Figure 19] Figure 19 is a graph (anti-BGP E4-Fab/FITC-K4C-TMR) showing the relationship between an antigen concentration in a human serum solution and a normalized fluorescence peak ratio (fluorescence peak of TAMRA/fluorescence peak of fluorescein).
[Figure 20] Figure 20 is a graph (anti-BGP E4-Fab/FITC-K4C-TMR) showing the relationship between an antigen concentration in a human serum solution and normalized fluorescence intensity (TAMRA).
[Figure 21] Figure 21 is a graph (anti-BGP E4-Fab/FITC-K4C-TMR) showing the relationship between an antigen concentration under conditions differing in probe concentration and fluorescence intensity (TAMRA).
[Figure 22] Figure 22 is a graph (anti-BGP E4-Fab/FITC-K4C-TMR) showing the relationship between an antigen concentration under conditions differing in probe concentration and a fluorescence peak ratio (fluorescence peak of TAMRA/fluorescence peak of fluorescein).

### [Description of Embodiments]

Hereinafter, the present invention will be described in detail.

### (A) Kit for antigen detection

The kit for antigen detection of the present invention is a kit for antigen detection, comprising: a first peptide conjugated with two fluorescent dyes capable of forming a FRET (fluorescence resonance energy transfer) pair or a fluorescent dye and a luminescent material capable of forming a BRET (bioluminescence resonance energy transfer) pair, and an antibody fragment with a second peptide added thereto, wherein 1) one of the first peptide and the second peptide is positively charged, and the other peptide is negatively charged, and 2) the first peptide and the second peptide are capable of forming a coiled coil.

As used herein, the "detection" means both of qualitative detection and quantitative detection.

One of the first peptide and the second peptide can be positively charged, and the other peptide can be negatively charged. It is preferred that the first peptide be positively charged and the second peptide be negatively charged.

The first peptide and the second peptide can be capable of forming a coiled coil. For example, K peptide and E peptide (Litowski et al., J. Biol. Chem. 277, 37272-9 (2002); and Yoshiaki Yano, Seibutsu Butsuri (journal of the Biophysical Society of Japan), 54, 323-324, 2014) mentioned above as well as c-Jun and c-Fos (Kd: 54 nM, Kohler, J.J. and Schepartz, A. Biochemistry, 40, 130-142, 2001), or LZA and LZB (Kd: 30 nM, O'Shea, E.K., Lumb, K.J. and Kim, P.S. Curr. Biol., 3, 658-667, 1993) can be used.

Specific examples of the first peptide can include a peptide comprising repeats of an amino acid sequence: X1-X2-X3-X4-X5-X6-X7 (wherein X1 and X6 each represent a positively charged amino acid, X7 represents a negatively charged amino acid, X2 and X5 each represent a hydrophobic amino acid, and X3 and X4 each represent any amino acid). In this context, the positively charged amino acid is, for example, lysine, arginine, or histidine, preferably lysine. The negatively charged amino acid is, for example, aspartic acid or glutamic acid, preferably glutamic acid. The hydrophobic amino acid is glycine, alanine, valine, proline, leucine, isoleucine, phenylalanine, tryptophan, or methionine, preferably leucine or isoleucine. The term "any amino acid" refers to, for example, glycine, alanine, valine, proline, leucine, isoleucine, phenylalanine, tryptophan, methionine, serine, threonine, asparagine, glutamine, tyrosine, cysteine, aspartic acid, glutamic acid, lysine, arginine, or histidine. Specific examples of the amino acid sequence: X1-X2-X3-X4-X5-X6-X7 can include Lys Ile Ala Ala Leu Lys Glu (SEQ ID NO: 3). The number of repeats of the amino acid sequence: X1-X2-X3-X4-X5-X6-X7 in the first peptide is not particularly limited. The number of repeats can be 2 to 6 and can be 3 to 5. The amino acid sequence: X1-X2-X3-X4-X5-X6-X7 is not necessarily required to start with X1 in the first peptide and may start with any of X2 to X7. This amino acid sequence is not necessarily required to end with X7 and may end with any of X1 to X6.

Specific examples of the first peptide can preferably include a peptide consisting of the amino acid sequence as set forth in SEQ ID NO: 1.

Specific examples of the second peptide can include a peptide comprising repeats of an amino acid sequence: X8-X9-X10-X11-X12-X13-X14 (wherein X8 and X13 each represent a negatively charged amino acid, X14 represents a positively charged amino acid, X9 and X12 each represent a hydrophobic amino acid, and X10 and X11 each represent any amino acid). In this context, the negatively charged amino acid is, for example, aspartic acid or glutamic acid, preferably glutamic acid. The positively charged amino acid is, for example, lysine, arginine, or histidine, preferably lysine. The hydrophobic amino acid is glycine, alanine, valine, proline, leucine, isoleucine, phenylalanine, tryptophan, or methionine, preferably leucine or isoleucine. The term "any amino acid" refers to, for example, glycine, alanine, valine, proline, leucine, isoleucine, phenylalanine, tryptophan, methionine, serine, threonine, asparagine, glutamine, tyrosine, cysteine, aspartic acid, glutamic acid, lysine, arginine, or histidine. Specific examples of the amino acid sequence: X8-X9-X10-X11-X12-X13-X14 can include Glu Ile Ala Ala Leu Glu Lys (SEQ ID NO: 4). The number of repeats of the amino acid sequence: X8-X9-X10-X11-X12-X13-X14 in the second peptide is not particularly limited. The number of repeats can be 2 to 6 and can be 3 to 5. The amino acid sequence: X8-X9-X10-X11-X12-X13-X14 is not necessarily required to start with X8 in the second peptide and may start with any of X9 to X14. This amino acid sequence is not necessarily required to end with X14 and may end with any of X8 to X13.

Specific examples of the second peptide can preferably include a peptide consisting of the amino acid sequence as set forth in SEQ ID NO: 2.

As used herein, the terms "positively charged" and "negatively charged" mean "positively charged" and "negatively charged" at physiological pH (e.g., pH 5 to 7) .

The two fluorescent dyes to be conjugated with the first peptide form a FRET pair. Specifically, one of the fluorescent dyes serves as a donor, and the other fluorescent dye serves as an acceptor.

The fluorescent dye serving as an acceptor is not particularly limited as long as the fluorescent dye permits quenching by interaction with the antibody fragment and dequenching by the addition of an antigen. Examples of such a fluorescent dye can include fluorescent dyes conventionally used in Q-body, for example, fluorescent dyes described in the specification of International Publication No. WO 2013/065314. Specific examples thereof can include fluorescent dyes having a backbone such as rhodamine, coumarin, Cy, EvoBlue, oxazine, carbopyronine, naphthalene, biphenyl, anthracene, phenanthrene, pyrene, or carbazole, and derivatives of these fluorescent dyes. Specific examples of the fluorescent dye can include CR110:carboxyrhodamine 110:Rhodamine Green (trade name), TAMRA:carbocytetremethlrhodamine:TMR, Carboxyrhodamine 6G:CR6G, ATTO655 (trade name), BODIPY FL (trade name):4,4-difluoro-5,7-dimethyl-4-bora-3a,4a-diaza-s-indancene-3-propionic acid, BODIPY 493/503 (trade name):4,4-difluoro-1,3,5,7-tetramethyl-4-bora-3a,4a-diaza-s-indancene-8-propionicacid, BODIPY R6G (trade name):4,4-difluoro-5-(4-phenyl-1,3-butadienyl)-4-bora-3a,4a-diaza-s-indancene-3-propionic acid, BODIPY 558/568 (trade name):4,4-difluoro-5-(2-thienyl)-4-bora-3a,4a-diaza-s-indancene-3-propionic acid, BODIPY 564/570 (trade name):4,4-difluoro-5-styryl-4-bora-3a,4a-diaza-s-indancene-3-propionic acid, BODIPY 576/589 (trade name):4,4-difluoro-5-(2-pyrrolyl)-4-bora-3a,4a-diaza-s-indancene-3-propionic acid, BODIPY 581/591 (trade name):4,4-difluoro-5-(4-phenyl-1, 3-butadienyl)-4-bora-3a,4a-diaza-s-indancene-3-propionic acid, Cy3 (trade name), Cy3B (trade name), Cy3.5 (trade name), Cy5 (trade name), Cy5.5 (trade name), EvoBlue10 (trade name), EvoBlue30 (trade name), MR121, ATTO 390 (trade name), ATTO 425 (trade name), ATTO 465 (trade name), ATTO488 (trade name), ATTO 495 (trade name), ATTO 520 (trade name), ATTO 532 (trade name), ATTO Rho6G (trade name), ATTO 550 (trade name), ATTO 565 (trade name), ATTO Rho3B (trade name), ATTO Rho11 (trade name), ATTO Rho12 (trade name), ATTO Thio12 (trade name), ATTO 610 (trade name), ATTO 611X (trade name), ATTO 620 (trade name), ATTO Rho14 (trade name), ATTO 633 (trade name), ATTO 647 (trade name), ATTO 647N (trade name), ATTO 655 (trade name), ATTO Oxa12 (trade name), ATTO 700 (trade name), ATTO 725 (trade name), ATTO 740 (trade name), Alexa Fluor 350 (trade name), Alexa Fluor 405 (trade name), Alexa Fluor 430 (trade name), Alexa Fluor 488 (trade name), Alexa Fluor 532 (trade name), Alexa Fluor 546 (trade name), Alexa Fluor 555 (trade name), Alexa Fluor 568 (trade name), Alexa Fluor 594 (trade name), Alexa Fluor 633 (trade name), Alexa Fluor 647 (trade name), Alexa Fluor 680 (trade name), Alexa Fluor 700 (trade name), Alexa Fluor 750 (trade name), Alexa Fluor 790 (trade name), Rhodamine Red-X (trade name), Texas Red-X (trade name), 5(6)-TAMRA-X (trade name), 5TAMRA (trade name), and SFX (trade name). Among them, particularly preferred examples of the fluorescent dye can include the rhodamine-based fluorescent dyes CR110 and TAMRA, and the oxazine-based fluorescent dye ATTO655.

The fluorescent dye serving as a donor can be capable of causing FRET with the fluorescent dye serving as an acceptor and can be appropriately selected according to the type of the fluorescent dye serving as an acceptor. When the fluorescent dye serving as an acceptor is, for example, a rhodamine-based fluorescent dye such as TAMRA, a fluorescein-based fluorescent dye such as FITC can be used as the fluorescent dye serving as a donor.

Examples of the combination of the fluorescent dye serving as an acceptor and the fluorescent dye serving as a donor can include TAMRA and FITC as well as TAMRA and AF488 (Rhodamine Green, Oregon Green, Tokyo Green), R6G and DAPI, ATTO655 and TAMRA, and Cy5 and TAMRA.

The fluorescent dye serving as an acceptor may be attached to any position on the first peptide as long as the fluorescent dye permits quenching by interaction with the antibody fragment and dequenching by the addition of an antigen. The fluorescent dye serving as an acceptor can be attached to, for example, the side chain of an amino acid residue, the N terminus, or the C terminus of the first peptide. The fluorescent dye serving as a donor may be attached to any position on the first peptide as long as the fluorescent dye is capable of causing FRET with the fluorescent dye serving as an acceptor. The fluorescent dye serving as a donor can be attached to, for example, the side chain of an amino acid residue, the N terminus, or the C terminus of the first peptide. In Examples mentioned later, the fluorescent dye serving as an acceptor (TAMRA) was attached to Cys at position 29 of the first peptide (SEQ ID NO: 1), and the fluorescent dye serving as a donor was attached to the N terminus or Lys at position 13, though the positions are not limited thereto.

A method for conjugating the fluorescent dyes to the first peptide is not particularly limited and can be appropriately selected according to the types of the fluorescent dyes used. Some commercially available fluorescent dyes have a reactive group (e.g., maleimide and isothiocyanate) that can specifically label the terminus of a peptide or the side chain of an amino acid residue. Therefore, the fluorescent dyes can be conjugated to the first peptide through the use of such a reactive group.

In the present invention, a luminescent material may be used instead of the fluorescent dye serving as a donor, and a fluorescent dye and a luminescent material capable of forming a BRET pair may be conjugated instead of the two fluorescent dyes capable of forming a FRET pair to the first peptide. Luciferase, aequorin, or the like can be used as the luminescent material. For example, firefly-derived luciferase, luminous shrimp (e.g., *Oplophorus gracilirostris*)-derived luciferase, Renilla-derived luciferase, or *Pyrearinus termitilluminans-derived* luciferase can be used as the luciferase.

A peptide tag, a linker, or the like may be added to the first peptide, and the first peptide may undergo a modification such as phosphorylation or methylation. In this context, examples of the peptide tag can include ProX tag, FLAG tag, His tag, HA tag, Ni tag, and Cys tag. Examples of the linker can include (G₄S)₂₋₆, (DDAKK)₂₋₆, and (EAAAK)₂₋₆, which are repeat sequences of G₄S, DDAKK, or EAAAK.

The antibody fragment is not particularly limited as long as the antibody fragment can quench the fluorescent dye serving as an acceptor and can cancel the quenching by the addition of an antigen.

The antibody fragment preferably has both of a light chain variable region and a heavy chain variable region, though the antibody fragment is not always limited thereto. Specific examples of the antibody fragment can include Fab, F(ab')₂, and scFv (single-chain variable fragment) as well as camelid heavy chain antibody-derived VHH.

The antibody fragment with a second peptide added thereto can be prepared by a gene recombination technique, as in conventional Q-body. Specifically, DNA containing a nucleotide sequence encoding the antibody fragment and a nucleotide sequence encoding the second peptide is transferred to an expression vector to prepare a recombinant vector. The antibody fragment with the second peptide added thereto can be expressed in an expression system using a host such as bacterial, yeast, insect, animal, or plant cells, or in a cell-free translation system. Conventional Q-body is prepared by preparing a Cys-tagged antibody fragment by a gene recombination technique, and adding a maleimide fluorescent dye thereto. However, use of the Cys tag might incur the misfolding of the antibody fragment or raises concerns about the cleavage of a disulfide bond within the antibody fragment in reduction reaction before fluorescent dye addition. A large feature of the present invention is that the fluorescent dye can be added to the antibody fragment without the use of such Cys tag.

The antigen is not particularly limited as long as the antigen is specifically recognized by the antibody fragment used. Examples thereof can include proteins, peptides, carbohydrates, lipids, glycolipids, and low-molecular compounds as well as protein modifications such as phosphorylation and methylation, and proteins that have undergone these modifications. The kit for antigen detection of the present invention is superior in detection sensitivity to immunoassay, such as ELISA, based on general competition principles and is therefore particularly useful in the detection of low-molecular compounds.

The kit for antigen detection of the present invention may comprise other constituents in addition to the first peptide conjugated with two fluorescent dyes capable of forming a FRET pair or a fluorescent dye and a luminescent material capable of forming a BRET pair and the antibody fragment with a second peptide added thereto. Examples of such other constituents can include antigens that can be used as standard substances, reagents for use in this kind of immunoassay kit, equipment, and instruction manuals.

The first peptide conjugated with two fluorescent dyes capable of forming a FRET pair or a fluorescent dye and a luminescent material capable of forming a BRET pair and the antibody fragment with a second peptide added thereto, which are contained in the kit for antigen detection of the present invention, may be conjugated with other substances, cells, or the like. For example, in a molecular display method, molecules such as proteins are bound to viral or microbial surface proteins and displayed on the surface of the viruses or the microbes. The antibody fragment with a second peptide added thereto may be displayed on the surface of a virus or a microbe.

The kit for antigen detection of the present invention has the following advantages.
1) The antigen can be simply detected from change in color without measuring fluorescence intensity.
2) Since the antigen can be detected from a fluorescence peak ratio between two colors, assay errors ascribable to errors of Q-body concentrations used can be reduced.
3) Fluorescence response is improved by antigen-dependent improvement in FRET efficiency resulting from the cancellation of acceptor dye quenching by the antigen as well as the acceptor dye which moves to the outside of the antibody by antigen addition and comes closer to the donor dye.
4) 488 nm laser can be used which is most commonly used in flow cytometry but cannot efficiently excite TAMRA.
5) The kit is capable of detecting the antigen even in serum and is thus useful in the field of clinical diagnosis.

### (B) Antigen detection method

The antigen detection method of the present invention is a method for detecting an antigen in a sample, comprising the steps of: (1) contacting the sample with the kit for antigen detection described above; and (2) detecting the fluorescence of the fluorescent dye(s) conjugated with the first peptide.

The sample can be any sample that has the possibility of containing the antigen to be detected, and may be a liquid sample or may be a non-liquid sample.

The liquid sample may be to be detected as it is or may be to be detected after being diluted with a buffer solution, physiological saline, or the like, or concentrated, or appropriately adjusted to pH, a salt concentration, etc., without impairing the antigen or inhibiting detection. Examples of such a liquid sample can include body fluids such as serum, plasma, saliva, spinal fluid, and urine, culture supernatants, cell extracts, bacterial cell extracts, and industrial waste water.

It is preferred that the non-liquid sample such as a solid be dissolved in, suspended in, or impregnated with a liquid such as a buffer solution or physiological saline and thereby put into a state that can be contacted with the kit for antigen detection described above, and then used as the sample. Before being dissolved in, suspended in, or impregnated with a liquid, the non-liquid sample may be subjected to a treatment such as divide, chopping, crushing, grinding, or slicing, or may be subjected to a treatment such as the removal or extraction of a particular component.

In the present invention, an *in vivo* body fluid such as blood or spinal fluid, tissues, or the like can also be used as the sample for detection. Specifically, the kit for antigen detection of the present invention is administered to a non-human animal such as a laboratory animal so that the antibody fragment can be contacted with an *in vivo* antigen. In this context, the non-human animal used can be an animal other than a human. Examples thereof can include non-human animals such as vertebrates, particularly, mammals, fish, bird, reptiles, and amphibians. Among them, a mammal is preferred, and a mouse, a rat, a hamster, a monkey, a pig, or the like is more preferred. The administration method is not particularly limited and can be appropriately selected from among parenteral local administration methods such as intramuscular injection, intraperitoneal injection, intravenous injection, subcutaneous injection, implantation, and application, and oral administration methods. An additional drug or the like may be administered at the same time with or before or after the kit for antigen detection of the present invention. The *in vivo* position or migration of the antigen, the amount of the antigen, or change therein may be observed by administering the kit for antigen detection of the present invention to a non-human animal. In such observation, a body fluid or tissues may be collected over time, and the measurement of fluorescence intensity thereof or the observation of fluorescence localization can be performed. Alternatively, *in vivo* fluorescence intensity, change therein, or the localization or migration of fluorescence can also be detected and observed in real time.

The contact of the sample with the kit for antigen detection may be performed under any condition and is usually performed in a liquid phase. Reaction conditions for the contact can be the same or similar conditions as in reaction using conventional Q-body. For example, reaction conditions described in International Publication No. WO 2013/065314 can be used. Specifically, the temperature condition can be, for example, 1 to 30°C, preferably 18 to 25°C, and the reaction time can be, for example, fraction of an instant to 180 minutes, preferably 1 to 90 minutes. In the case of performing reaction in the body of a non-human animal, the administered kit is incubated for, for example, 5 to 180 minutes, preferably 60 to 120 minutes, and, if necessary, a treatment such as the harvest of tissues, blood, cells, or the like, or the exposure of a site to be observed can be appropriately performed.

When the first peptide conjugated with fluorescent dyes and the antibody fragment with a second peptide added thereto are mixed, the first peptide and the second peptide bind to each other by forming a coiled coil to produce an antibody fragment labeled with two fluorescent dyes (donor and acceptor) (FRET CQ-body). In the absence of the antigen to be detected in the sample, the fluorescent dye serving as an acceptor is in a state incorporated inside the antibody fragment. In this state, the fluorescent dye serving as an acceptor is quenched by an amino acid in the antibody fragment. Also, in this state, the fluorescent dye serving as an acceptor is located distant from the fluorescent dye serving as a donor. Therefore, FRET efficiency is low. Hence, the fluorescent dye serving as a donor emits strong fluorescence by irradiation with excitation light for the fluorescent dye serving as a donor, whereas the fluorescent dye serving as an acceptor rarely emits fluorescence. In contrast to this, in the presence of the antigen to be detected in the sample, the fluorescent dye serving as an acceptor moves to the outside of the antibody fragment so that the quenching is canceled. By this movement, the fluorescent dye serving as an acceptor comes closer to the fluorescent dye serving as a donor and thereby causes FRET. Hence, the fluorescent dye serving as an acceptor emits strong fluorescence by irradiation with excitation light for the fluorescent dye serving as a donor, whereas the fluorescent dye serving as a donor rarely emits fluorescence. Thus, the method of the present invention enables the presence or absence of the antigen to be determined from the color of fluorescence (whether the fluorescent dye serving as an acceptor emits strong fluorescence or the fluorescent dye serving as a donor emits strong fluorescence), and can conveniently detect the antigen. Furthermore, the method of the present invention can detect the antigen directly without going through a washing step, as described above. Although the case of using a fluorescent dye as a donor is described above, the presence or absence of the antigen can also be determined from the color of light, as in the above, in the case of using a luminescent material instead of the fluorescent dye.

In the method of the present invention, irradiation with excitation light for the fluorescent dye serving as a donor is usually performed, and the antigen is detected on the basis of the color of fluorescence (wavelength of fluorescence) resulting therefrom. The excitation light to be used in irradiation can be appropriately selected according to the type of the fluorescent dye serving as a donor. In the case of using, for example, FITC as the fluorescent dye serving as a donor, the wavelength of the excitation light can be around 485 nm. When the donor is a luminescent material, not the fluorescent dye, irradiation with excitation light is not necessary. A substance necessary for luminescence, for example, a luminescent substrate or ATP, is added instead.

In the method of the present invention, as described above, the presence or absence of the antigen can be determined from the color of fluorescence. Therefore, the measurement of fluorescence intensity using measurement equipment is not necessarily required. However, the fluorescence intensity of the fluorescent dye serving as a donor and/or the fluorescence intensity of the fluorescent dye serving as an acceptor may be measured. In this case, in the presence of the antigen in the sample, the fluorescence intensity of the fluorescent dye serving as a donor is decreased and the fluorescence intensity of the fluorescent dye serving as an acceptor is increased, as compared with a control (sample free from the antigen). Therefore, the presence or absence of the antigen can be determined on the basis of these. The presence or absence of the antigen can be more accurately determined by determining the ratio between the fluorescence intensity of the fluorescent dye serving as a donor and the fluorescence intensity of the fluorescent dye serving as an acceptor. The wavelength of the fluorescence to be measured can be appropriately selected according to the type of the fluorescent dye used. In the case of using, for example, FITC as the fluorescent dye serving as a donor, fluorescence around 520 nm is measured. In the case of using TAMRA as the fluorescent dye serving as an acceptor, fluorescence around 580 nm is measured.

Conversely, TAMRA or the like may be introduced as the donor dye to a position that facilitates quenching by an amino acid in the antibody fragment, in the first peptide, and a dye, such as Cy5, having a longer wavelength can be introduced as the acceptor dye to a position that hinders quenching, in the first peptide.

As a result, the presence or absence of the antigen can be determined from the cancellation of donor quenching by antigen addition and synergistic increase in the fluorescence intensity of the acceptor ascribable to enhanced FRET efficiency.

A light source or a measurement apparatus for use in fluorescence intensity measurement can be appropriately selected. The light source can permit irradiation at an excitation light wavelength. Examples of the light source can include mercury lamps, xenon lamps, LED, and laser beam. Excitation light with a particular wavelength can be obtained using an appropriate filter. A device usually used in fluorescence observation can be used as a fluorescence measurement apparatus, and a light source for excitation light and an irradiation system thereof, a microscope equipped with a fluorescence image capturing system, flow cytometry, or the like can be appropriately used. Examples thereof can include MF20/FluoroPoint-Light (manufactured by Olympus Corp.), FMBIO-III (manufactured by Hitachi Software Engineering Co., Ltd.), and SH-800 (manufactured by Sony Corp.). Since fluorescence intensity has positive correlation with the concentration of the antigen, the fluorescence intensity is measured using a sample containing the antigen having a known concentration to prepare a standard curve that indicates the relationship between the antigen concentration and the fluorescence intensity. The concentration of the antigen having an unknown concentration can be calculated from the standard curve. In the calculation of such an antigen concentration, the amount of the antigen may be automatically calculated according to a conversion equation or the like established on the basis of a standard curve prepared in advance. The measurement of fluorescence intensity may be the measurement of a fluorescence spectrum or may be the measurement of fluorescence intensity at a particular wavelength.

In the case of administering the kit for antigen detection of the present invention to a non-human animal, its body fluid, tissues, or the like may be collected. In addition, a region to be detected in the non-human animal may be irradiated with excitation light to detect the fluorescence of the fluorescent dyes. Examples of this case can include use of a fluorescence microscope, a fluorescence image analyzer, or an endoscope equipped with a light source. For detection, it is preferred to also obtain an image showing the structure of the individual, tissues, or cells of the non-human animal using an endoscope, X ray, CT, MRI, ultrasonic wave, a microscope, or the like. Since the fluorescence intensity of the fluorescent dye serving as an acceptor has positive correlation with the amount of the antigen, the localization (position) and/or amount of the antigen can be determined on the basis of a two-dimensional or three-dimensional image of the detected fluorescence. This result may be compared with the image showing the structure described above. For the detection of such fluorescence, it is preferred that a sample from which the kit for antigen detection of the present invention has been removed, a buffer solution used in the dilution of the sample, or the like be prepared as a negative control and also subjected to the fluorescence detection. Also, the amount of the antigen may be calculated, for example, using a fluorescence intensity ratio obtained by dividing a measurement value from the target sample by a measurement value from the negative control. Alternatively, since the fluorescence intensity of the fluorescent dye serving as an acceptor has positive correlation with the amount of the antigen, the presence of the antigen in the assay sample may be determined when fluorescence intensity that exceeds an appropriately set threshold is obtained.

As described above, the present invention allows detection of all antigens that can be assayed by immunoassay such as ELISA, immunodiffusion, latex agglutination assay, immunochromatography, or surface plasmon resonance. For example, competitive ELISA is generally used as immunoassay on low-molecular substances. The detection of low-molecular substances according to the present invention is superior in the convenience of an approach, assay sensitivity, SN ratio, etc. to competitive ELISA and can exerts its greatest ability. Examples of such a low-molecular compound suitable for the detection of the present invention can include: stimulant drugs or narcotic drugs such as amphetamine, methamphetamine, morphine, heroin, and codeine; mycotoxins such as aflatoxin, sterigmatocystin, neosolaniol, nivalenol, fumonisin, ochratoxin, and endophyte-producing toxins; sex hormones such as testosterone and estradiol; additives that are misapplied to feed, such as clenbuterol and ractopamine; harmful substances such as PCB, gossypol, histamine, benzpyrene, melamine, acrylamide, and dioxin; residual agricultural chemicals such as acetamiprid, imidacloprid, chlorfenapyr, malathion, carbaryl, clothianidin, triflumizole, chlorothalonil, spinosad, Lannate, methamidophos, and chlorpyrifos; and environmental hormones such as bisphenol A.

According to the present invention, measurements are instantly obtained, and in addition, a detection method is simple. This allows a smaller size and a lower price of equipment. These advantages are not limited to the detection of low-molecular substances and the strength of the invention can be demonstrated in onsite analysis for which analyzers visit the site and conduct assay. Furthermore, since the assay is easy, even persons other than specialists can conduct the assay. The present invention can show its ability to the fullest, for example, in the field of clinical diagnosis involving causative viruses or bacteria of infections such as influenza and contagious diseases, drug concentrations in blood, or POCT, the field of simple health checkup in workplaces, schools, nurseries, or homeplaces, security and safety fields such as anti-terrorism measures against anthrax, botulinum toxin, sarin, or VX gas, environmental fields for environmental pollutants or house dust which requires assay on the spot, and the field of research and development which require immunoassay.

### [Examples]

Hereinafter, the present invention will be described in more detail with reference to Examples. However, the present invention is not limited by these Examples.

### [Example 1] Q-body using anti-BGP E4-Fab/K2-FITC-K2C-TMR

### (1) Preparation of anti-BGP E4-Fab

A plasmid vector encoding a peptide (anti-BGP E4-Fab) having E4 peptide ((EIAALEK)₄) added to the N terminus of a heavy chain of a Fab fragment (anti-BGP-Fab) (Abe, R et al., Scientific Reports 4, 4640, 2014) that recognized osteocalcin (BGP) was constructed, and this protein was expressed using *E. coli* SHuffle T7 express lys Y and purified.

### (2) Preparation of K2-FITC-K2C-TMR and FITC-K4C-TMR

FITC was introduced to Lys at position 13 counted from the N terminus of K4 peptide ((KIAALKE)₄), and Cys was added to the C terminus thereof to prepare a peptide (K2-FITC-K2C). K4 peptide was N-terminally modified with FITC, and Cys was added to the C terminus thereof to prepare a peptide (FITC-K4C). These peptides were synthesized by commission synthesis (Lifetein, NJ, USA). These peptides were reacted with a fluorescent dye 5-TMR-C6-maleimide to introduce TMR to the C-terminally added Cys. The obtained modified peptides were purified by high-performance liquid chromatography using a reverse-phase column to obtain K2-FITC-K2C-TMR and FITC-K4C-TMR, respectively.

### (3) Preparation of FRET CQ-body

### (3-1) Materials

- Anti-BGP E4-Fab
- K2-FITC-K2C-TMR
- PBST (PBS + 0.1 % Tween 20)

### (3-2) Procedures

2.0 µL of 1.0 µM anti-BGP E4-Fab and 1.3 µL of 0.97 µM K2-FITC-K2C-TMR were mixed and left standing at room temperature for 10 minutes in the dark. Then, the mixture was suspended by the addition of 250 µL of PBST so that the concentrations of anti-BGP E4-Fab and K2-FITC-K2C-TMR were adjusted to 8.0 nM and 5 nM, respectively.

### (4) Fluorescence spectrum measurement

### (4-1) Equipment

- FP-8500 fluorospectrophotometer (JASCO Corp.)
- 5 × 5 mm quartz microcell (Starna Scientific, UK)

### (4-2) Conditions

Temperature: 25°C
Excitation band width: 5 nm
Fluorescence band width: 5 nm
Response: 2 sec
Sensitivity : High
Data capture interval: 0.5 nm
Scanning rate: 500 nm/min
Excitation wavelength: 485 nm
Measurement wavelength: 510 to 700 nm
Light source: xenon lamp

### (4-3) Procedures and results

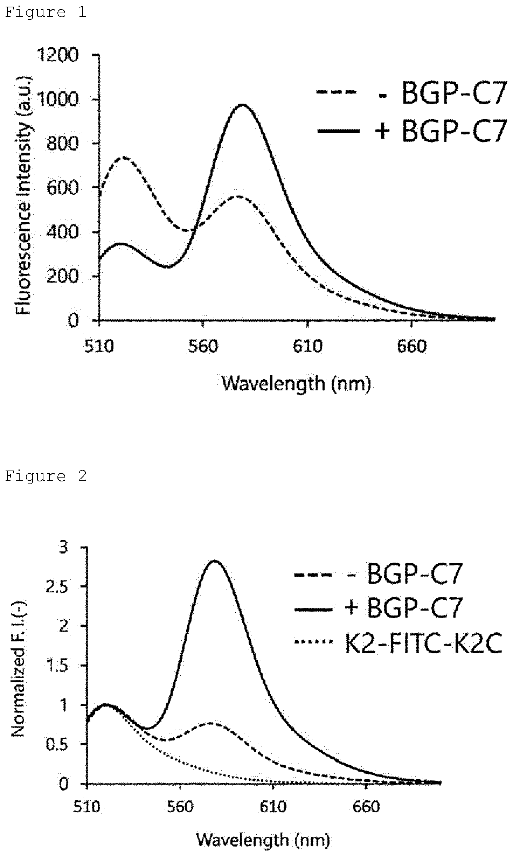

250 µL of the prepared FRET CQ-body was added to a quartz cell. At the same time therewith, a stirrer was placed in the microcell, and a solution during assay was homogenized by stirring. An 8 nM anti-BGP E4-Fab solution was provided as a blank in another cell. A 5 nM K2-FITC-K2C peptide solution was further provided in an alternative cell. These cells were loaded in an assay apparatus, and the blank was assayed, followed by sample assay. Then, 2.5 µL (final concentration: 1 µM) of 100 µM BGP-C7 was added as an antigen, and the solution was stirred for 5 minutes and left standing for 5 minutes to perform sample assay again. Figure 1 shows the fluorescence spectrum of FRET CQ-body before and after antigen addition.

### (5) Data processing

The spectrum of K2-FITC-K2C peptide was overlaid with the spectrum of Figure 1. As for the respective spectra of FRET CQ-body - BGP, FRET CQ-body + BGP, and K2-FITC-K2C peptide, a fluorescence intensity value in the whole wavelength range was divided by a fluorescence intensity value at the top of the spectrum in the range of 510 to 525 nm (normalization, Figure 2).

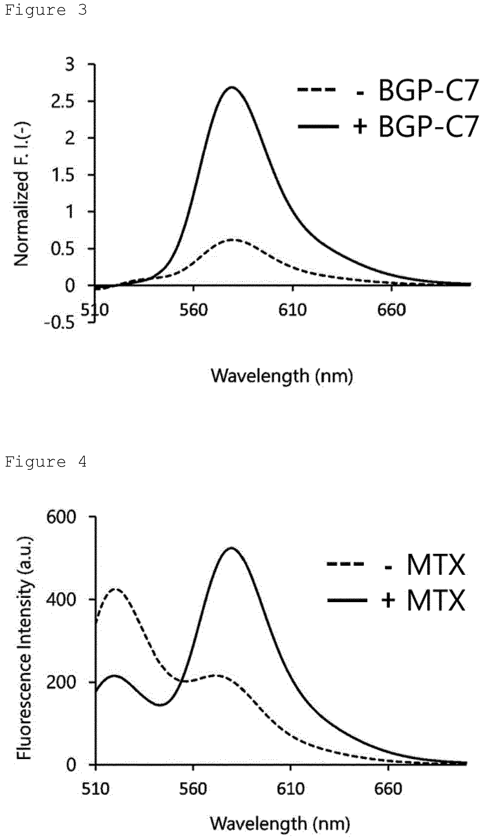

Finally, the fluorescence intensity value of K2-FITC-K2C peptide was subtracted from the respective fluorescence intensity values of FRET CQ-body - BGP and FRET CQ-body + BGP to obtain a net fluorescence response spectrum in antigen addition (Figure 3).

### [Example 2] Q-body using anti-MTX E4-VHH/K2-FITC-K2C-TMR

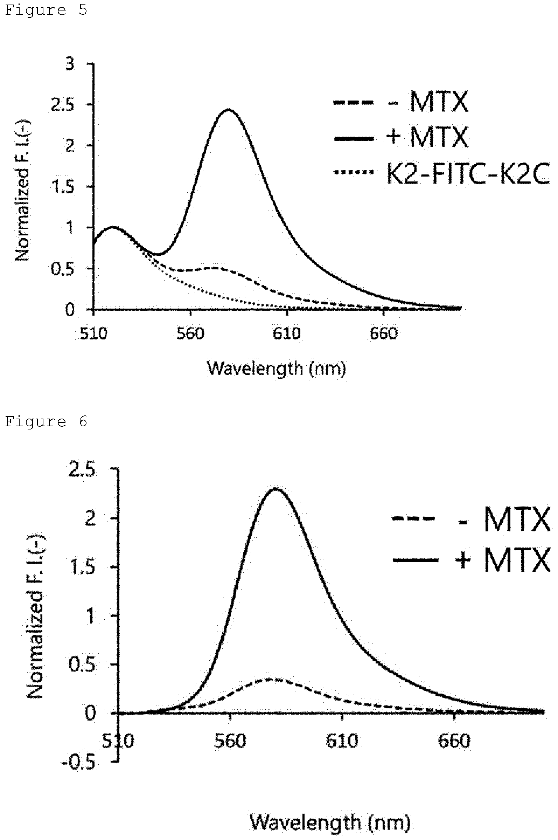

FRET CQ-body was prepared in the same manner as in Example 1 except that llama-derived heavy chain antibody fragment VHH (anti-MTX-VHH) (Fanning, SW. and Horn, JR. Protein Science 20, 1196-1207, 2011) that recognized an anticancer agent methotrexate (MTX) was used instead of the Fab fragment (anti-BGP-Fab) that recognized osteocalcin (BGP). Figure 4 shows the fluorescence spectrum of this FRET CQ-body before and after antigen addition. Figures 5 and 6 show spectra obtained by the same treatments as in Figures 2 and 3, respectively, in Example 1.

### [Example 3] Q-body using anti-BGP E4-Fab/FITC-K4C-TMR

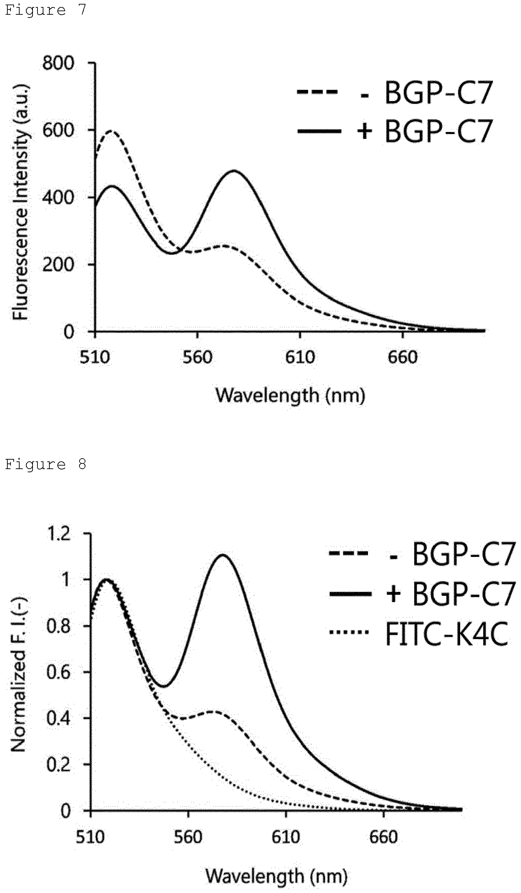

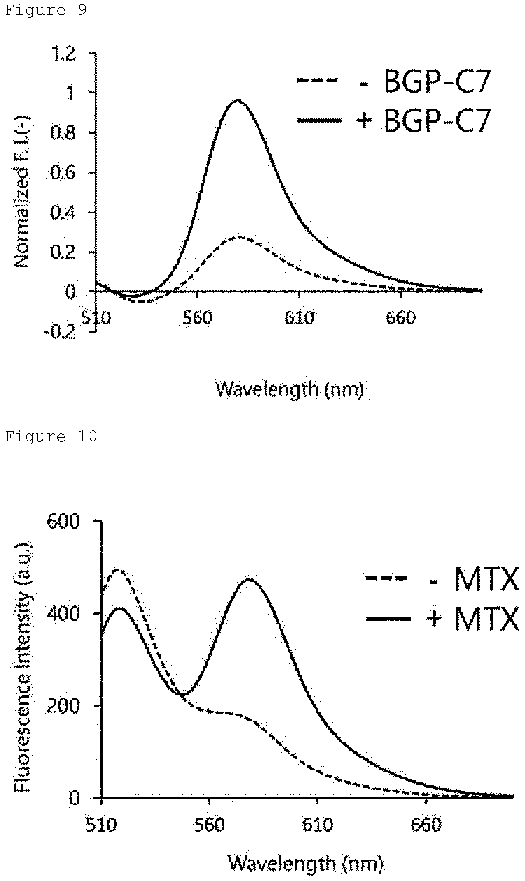

FRET CQ-body was prepared in the same manner as in Example 1 except that FITC-K4C-TMR was used in which FITC was introduced to the N terminus instead of introducing FITC to Lys at position 13 counted from the N terminus of K4 peptide and the C-terminal Cys was modified with TAMRA. Figure 7 shows the fluorescence spectrum of this FRET CQ-body before and after antigen addition. Figures 8 and 9 show spectra obtained by the same treatments as in Figures 2 and 3, respectively, in Example 1.

### [Example 4] Q-body using anti-MTX E4-VHH/ FITC-K4C-TMR

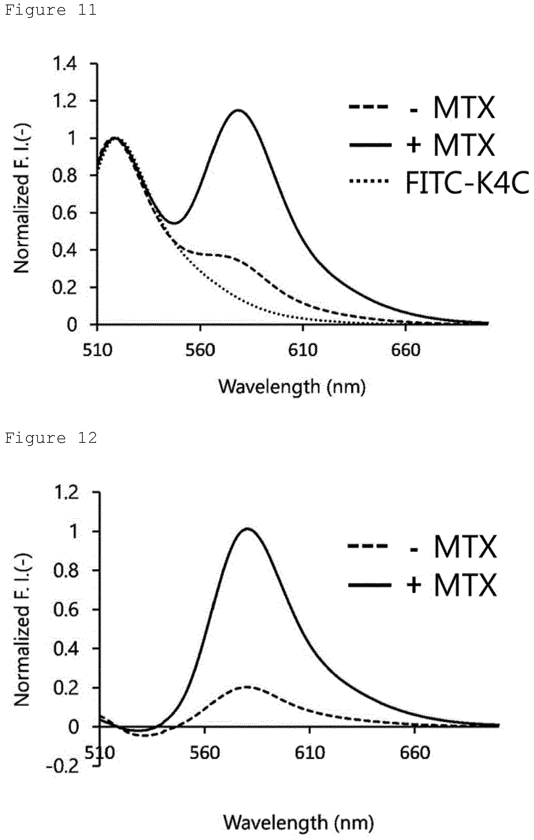

FRET CQ-body was prepared in the same manner as in Example 1 except that llama-derived heavy chain antibody fragment VHH (anti-MTX-VHH) that recognized an anticancer agent methotrexate (MTX) was used instead of the Fab fragment (anti-BGP-Fab) that recognized osteocalcin (BGP), and mixed with FITC-K4C-TMR at a molar ratio of 8:5. Figure 10 shows the fluorescence spectrum of this FRET CQ-body before and after antigen addition. Figures 11 and 12 show spectra obtained by the same treatments as in Figures 2 and 3, respectively, in Example 1.

### [Example 5] Evaluation of Q-body on yeast cell surface

### (1) Preparation of vector for VHH fragment with E4 added thereto display on yeast

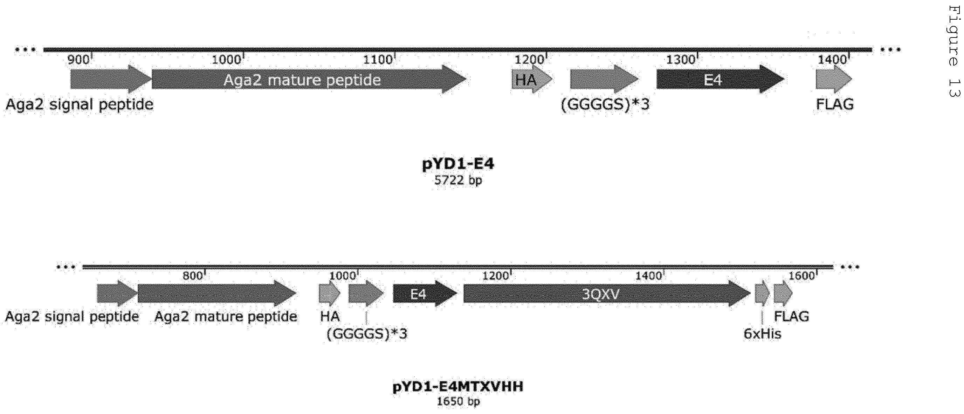

Provided that fluorescence response is measured by displaying an antibody fragment with E4 added thereto not only in a solution but also on yeast cell surface and can be selected according to performance as Q-body, it would be possible to select Q-body having high performance. Accordingly, PCR was performed according to a routine method using, as a template, a plasmid pEQ-VHH(MTX) having a BamHI cleavage site downstream of MTX-recognizing VHH used in Example 2 and using a primer NheI_E4back (TCAGCTAGCATGGCTGAAATCGCTGC) (SEQ ID NO: 5) and T7 terminator (ATGCTAGTTATTGCTCAGCGG) (SEQ ID NO: 6) to prepare an E4-VHH(MTX) fragment containing terminal NheI and BamHI cleavage sites. Also, an E4 fragment containing terminal NheI and BamHI cleavage sites was prepared by using two primers NheI_E4back and BamHI_E4for (CGCGGATCCCTGACCGGTGCCTCC) (SEQ ID NO: 7). Any of these fragments and a pYD1-derived plasmid pYD1-mSA (Lim et al. Biotechnology and Bioengineering, 2013, 39865, Addgene) for monomer streptavidin display on yeasts were each treated with restriction enzymes NheI and BamHI to prepare two types of plasmids in which Aga2 anchor protein gene was added to the N terminus of E4-VHH(MTX) or E4. Figure 13 shows the partial structures of the plasmids.

### (2) Transformation

EBY100 (ATCC(R) MYA-4941(TM)) was transformed with each of the two plasmids thus prepared using Frozen-EZ Yeast Transformation II Kit (Zymo Research Corp.) according to manufacturer's instruction.

### (3) Expression induction

### (3-1) Materials

- SD agar medium (26.7 g/L minimal SD base, 0.72 g/L dropout (DO) supplement (-Trp,-Ura), 2 % d-glucose, 1.5 % agar, 50 µg/ml ampicillin)
- SD liquid medium (26.7 g/L minimal SD base, 0.72 g/L DO supplement (-Trp,-Ura), 2 % glucose, 100 mM phosphate buffer pH 6, 50 µg/ml ampicillin)
- SG liquid medium (26.7 g of minimal SD base, 0.72 g of DO supplement (-Trp,-Ura), 2 % galactose, 100 mM phosphate buffer pH 6, 50 µg/ml ampicillin)

### (3-2) Procedures

The transformants thus obtained were screened in an SD agar medium. Then, the formed colony was added to 10 ml of an SD liquid medium and cultured overnight at 30°C at 250 rpm. On the next day, OD₆₀₀ was measured, and the cultures were diluted with an SD liquid medium and cultured in 20 ml of an SD liquid medium at OD₆₀₀ of 0.2 until OD₆₀₀ became 0.4 to 0.8. Then, the supernatant was removed by centrifugation at 2,500 g at 4°C for 5 minutes. Then, 20 ml of an SG liquid medium was added to the cells, which were then cultured at 250 rpm at 20°C for 48 hours.

### (4) Fluorescence measurement of E4 displayed on yeast cell surface

### (4-1) Materials

- Yeast cell sample allowed to express E4 in (3)
- K4-TAMRA peptide
- FITC-K4 peptide
- K2-FITC-K2 peptide
- FITC-K4-TAMRA peptide
- K2-FITC-K2-TAMRA peptide
- BPBS (PBS + 0.1 % BSA)

### (4-2) Equipment

- Flow Cytometry (FACS) SH-800 with cell sorter functions (manufactured by Sony Corp.)

### (4-3) Sample preparation

After expression induction, OD₆₀₀ of the culture solution was measured to estimate the number of cells in the culture solution (OD₆₀₀ = 1 = 1.0 × 10⁷ cells/ml). Then, the culture solution containing 1.0 × 10⁷ cells was aliquoted into 5 samples. The supernatant was removed by centrifugation at 14000 g at 4°C for 1 minute. The cells were washed with 2 ml of BPBS and then centrifuged again at 14000 g at 4°C for 1 minute, followed by the removal of the supernatant to adjust the volume to 50 µl. K4-TAMRA, FITC-K4, K2-FITC-K2, FITC-K4-TAMRA, and K2-FITC-K2-TAMRA prepared in Example 1 were each added thereto at a final concentration of 1 µM, and further 50 µl of BPBS was added to adjust the volume to 100 µl. The mixture was left standing on ice for 30 minutes. Operation of centrifuging the resultant at 14000 g at 4°C for 1 minute for the removal of the supernatant, and adding 1 ml of BPBS, followed by stirring was repeated twice to remove unbound peptides. Then, 500 µl of BPBS was added thereto to prepare a sample solution.

### (4-4) Assay conditions

Laser (488 nm) was used, and FSC, BSC, FITC, and PE (for TAMRA detection) were used as detectors. However, the sensitivity of the detectors was adjusted with the software of SH-800 to FSC (13), BSC (37.5%), FITC (39.5%), and PE (42.5%), respectively.

### (4-5) Compensation of fluorescence intensity

In order to compensate for the leakage of FITC into the PE detector and the leakage of TAMRA into the FITC detector, samples labeled with K4-TAMRA, FITC-K4, or K2-FITC-K2 were used in the compensation according to the software of SH-800. In the following assay and results, results obtained after the compensation on the basis of the results obtained here will be shown.

### (4-6) Assay and results

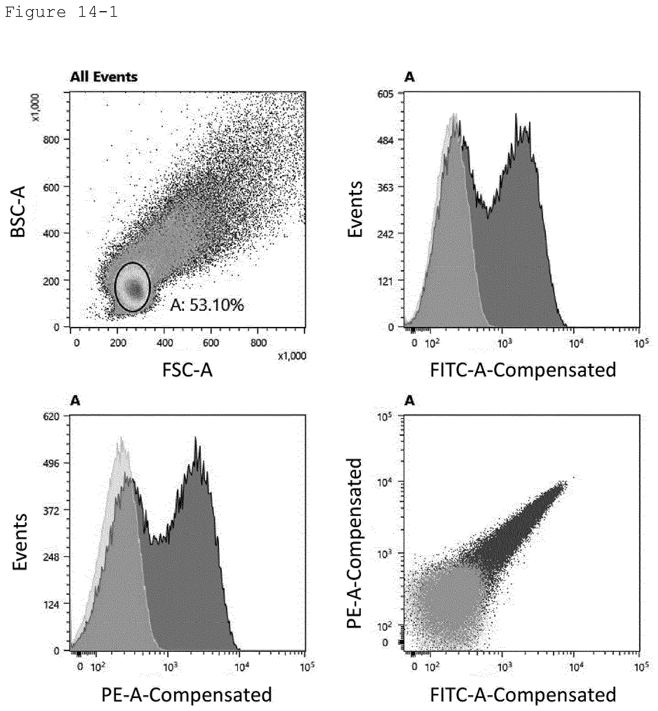

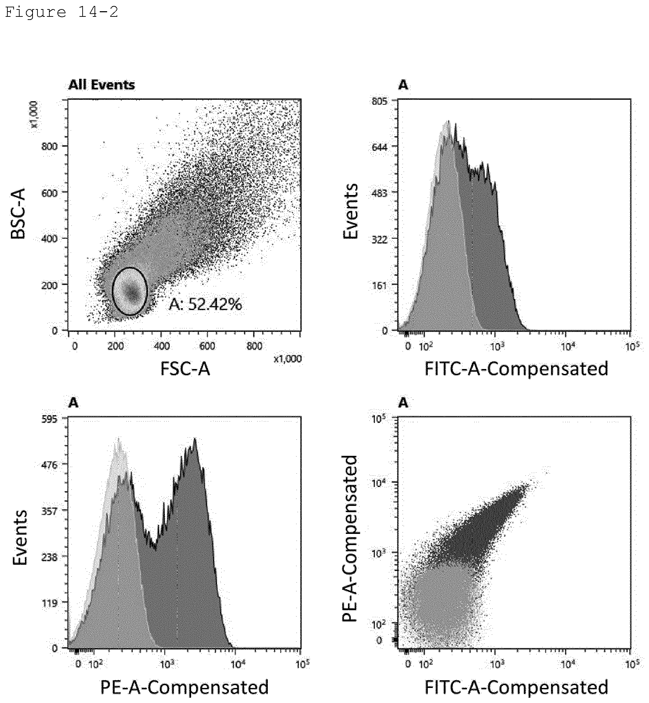

The samples labeled with FITC-K4-TAMRA or K2-FITC-K2-TAMRA were assayed by FACS to prepare histograms for 100,000 cells (Figures 14-1 and 14-2). As a result of measuring the fluorescence intensity distributions of the cell populations indicated by A in the upper left graphs, it was found that in all the cells, TAMRA-derived fluorescence (PE-A-Compensated) was able to be detected with sufficient intensity as compared with the fluorescence of unlabeled samples indicated by light gray.

### (5) Performance evaluation of Q-body on yeast cell surface

### (5-1) Materials

- Yeast cell sample allowed to express E4-VHH(MTX) in (3)
- FITC-K4-TAMRA peptide
- K2-FITC-K2-TAMRA peptide
- BPBS

### (5-2) Equipment

- SH-800 (manufactured by Sony Corp.)

### (5-3) Sample preparation

After expression induction, OD₆₀₀ of the culture solution was measured to estimate the number of cells in the culture solution (OD₆₀₀ = 1 = 1.0 × 10⁷ cells/ml). Then, the culture solution containing 2.0 × 10⁷ cells was aliquoted into 2 samples. The supernatant was removed by centrifugation at 14000 g at 4°C for 1 minute. The cells were washed with 2 ml of BPBS and then centrifuged again at 14000 g at 4°C for 1 minute, followed by the removal of the supernatant to adjust the volume to 50 µl. FITC-K4-TAMRA and K2-FITC-K2-TAMRA prepared in Examples were each added thereto at a final concentration of 1 µM, and further 50 µl of BPBS was added to adjust the volume to 100 µl. The mixture was left standing on ice for 30 minutes. Operation of centrifuging the resultant at 14000 g at 4°C for 1 minute for the removal of the supernatant, and adding 1 ml of BPBS, followed by stirring was repeated twice to remove unbound peptides. Then, 1 ml of BPBS was added thereto, and the mixture was stirred and divided into 500 µl aliquots. Methotrexate (MTX) was added as an antigen at a final concentration of 1 µM to one of the aliquots.

### (5-4) Assay conditions

The same conditions as in (4-4) were used.

### (5-5) Compensation of fluorescence intensity

The same conditions as in (4-5) were used.

### (5-6) Assay and results

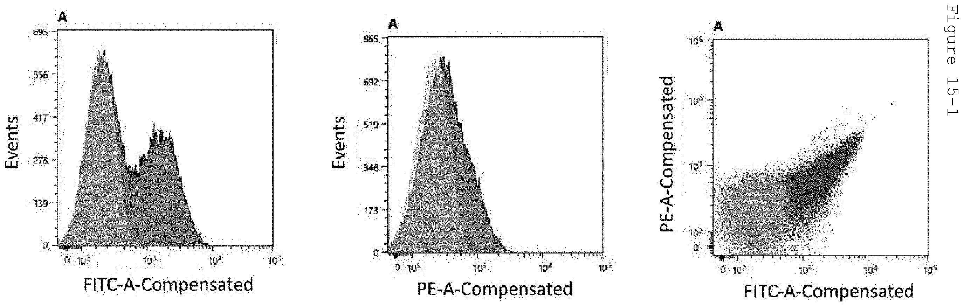

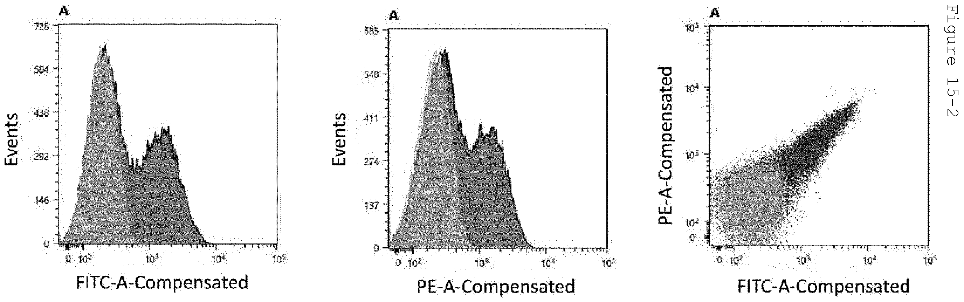

The samples labeled with FITC-K4-TAMRA were assayed by FACS in the absence (Figure 15-1) and the presence (Figure 15-2) of the antigen MTX to prepare histograms for 100,000 cells. The fluorescence of unlabeled samples was indicated by light gray on the histograms. As a result, it was revealed that TAMRA-derived fluorescence intensity (PE-A-Compensated) lower than that in Figure 14-1 without the antigen was markedly increased by the addition of the antigen. By contrast, no particular change was seen in FITC-derived fluorescence intensity.

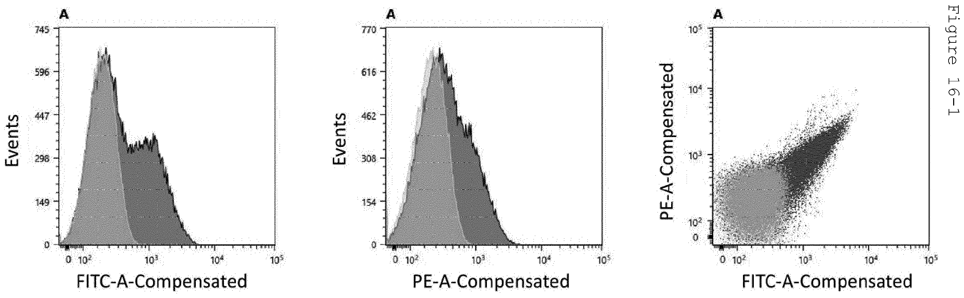

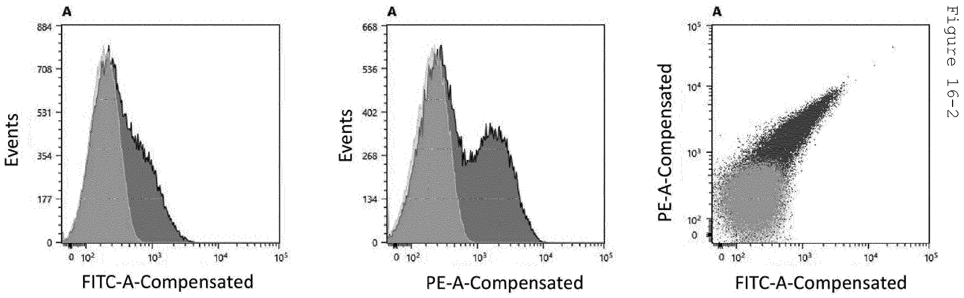

The samples labeled with K2-FITC-K2-TAMRA were assayed by FACS in the absence (Figure 16-1) and the presence (Figure 16-2) of MTX to prepare histograms for 100,000 cells. As a result, it was revealed that TAMRA-derived fluorescence intensity (PE-A-Compensated), which was low without the antigen, was markedly increased by the addition of the antigen. Furthermore, FITC-derived fluorescence intensity was slightly decreased by the addition of the antigen. As in the solution, it was considered that FITC-derived fluorescence was weakened while the fluorescence of TAMRA was potentiated, because TAMRA that moved from an antigen binding site came closer to FITC by antigen addition so that FRET efficiency was enhanced. These results demonstrated that use of FACS allows observation and screening of quenching and antigen-dependent dequenching of anti-MTX E4 VHH Q-body expressed on yeast cell surface.

### [Example 6] Preparation of calibration curve of BGP using anti-BGP E4-Fab/FITC-K4C-TMR

### (1) Preparation of FRET CQ-body

### (1-1) Materials

- Anti-BGP E4-Fab (the same as that used in Example 1 was used.)
- FITC-K4C-TMR (the same as that used in Example 3 was used.)
- BPBST (PBS + 0.1 % Tween 20 + 0.1 % BSA)

### (1-2) Procedures

2.0 µL of 5.0 µM anti-BGP E4-Fab and 5.8 µL of 1.7 µM FITC-K4C-TMR were mixed, and 2.2 µL of BPBST was added thereto to adjust their respective final concentrations to 1.0 µM. The mixture was left standing at room temperature for 10 minutes in the dark. Then, 5 µL of the reaction solution was collected into a 1.5 mL tube and suspended by the addition of 1.0 mL of BPBST so that the concentrations of E4-Fab and FITC-K4C-TMR were each adjusted to 5.0 nM.

### (2) Fluorescence spectrum measurement

### (2-1) Equipment

The same equipment as that used in Example 1 was used.

### (2-2) Conditions

The same conditions as in Example 1 were used.

### (2-3) Procedures and results

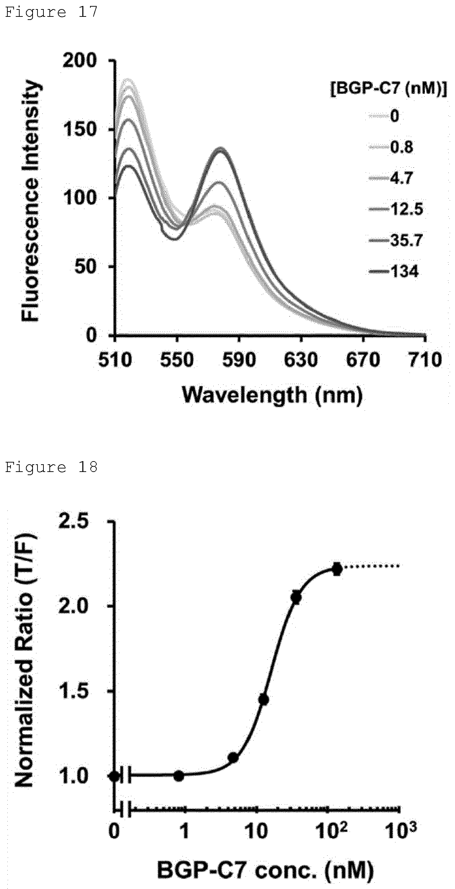

250 µL of BPBST was provided in a quartz cell and assayed as a blank. Subsequently, 250 µL of the prepared FRET CQ-body was added to each of three quartz cells. At the same time therewith, a stirrer was placed in each microcell, and a solution during assay was homogenized by stirring. After stirring at room temperature for 20 minutes, these samples with 0 nM antigen were assayed. Then, 2.0 µL of 0.1 µM BGP-C7 was added as an antigen to each cell. After stirring at room temperature for 15 minutes, these samples with 0.8 nM antigen were assayed. Thereafter, the addition of the antigen, stirring at room temperature for 15 minutes, and sample assay were performed by the same procedures as above to obtain results of measuring a fluorescence spectrum at antigen concentrations of 4.7, 12.5, 35.7, and 134 nM (Figure 17) .

### (3) Data processing

The ratio between the fluorescence peak of TAMRA (maximum value in a zone from 551 to 710 nm) and the fluorescence peak of fluorescein (maximum value in a zone from 510 to 550 nm) (TAMRA/fluorescein) at each antigen concentration was taken for each of 3 measurements, and a mean and standard deviation were calculated (n = 3). Subsequently, the fluorescence peak values at all the antigen concentrations were divided by the value of the fluorescence peak ratio at the antigen concentration of 0 nM (normalization). As a result of curve fitting using a 4-parameter logistic curve as a regression model, EC₅₀ was 16 nM and a hill coefficient was 2.1. A detection limit concentration determined by the 3σ method was 2.0 nM. Figure 18 shows a graph in which the abscissa of the obtained regression curve was expressed on a logarithmic scale.

### [Example 7] Preparation of calibration curve of BGP in human serum using anti-BGP E4-Fab/FITC-K4C-TMR

### (1) Preparation of FRET CQ-body

### (1-1) Materials

- Anti-BGP E4-Fab (the same as that used in Example 1 was used.)
- FITC-K4C-TMR (the same as that used in Example 3 was used.)
- PBS
- BPBST (PBS + 0.1 % Tween 20 + 0.1 % BSA)
- HUMAN POOLED SERUM (MP Biomedicals, LLC, Cat# 2931149)

### (1-2) Procedures

5.0 µL of 5.0 µM anti-BGP E4-Fab and 13 µL of 1.7 µM FITC-K4C-TMR were mixed, and 7.0 µL of BPBST was added thereto to adjust their respective final concentrations to 1.0 and 0.9 µM. The mixture was left standing at room temperature for 10 minutes in the dark. Then, the reaction solution was suspended by the addition of 2.5 mL of human serum diluted into 20% with PBS so that the concentrations of E4-Fab and FITC-K4C-TMR were adjusted to 10 nM and 8.8 nM, respectively.

### (2) Fluorescence intensity measurement

### (2-1) Equipment

- Multimode microplate reader CLARIOstar (BMG LABTECH)
- Microplate 96 well, PS, Half area, Black, High binding, Sterile (Greiner Bio-One, Cat# 675077)

### (2-2) Conditions

Common
Gain: 1500
Focal height: 8.0 mm
Target value: 1%
Single-wavelength measurement
Ex: 541.2 ± 14
Dichroic: 562.5
Em: 580 ± 13
Two-wavelength measurement

For FITC
Ex: 480 ± 20
Dichroic: 502.5
Em: 530 ± 30
For TAMRA
Ex: 480 ± 20
Dichroic: auto 530
Em: 585 ± 30

### (2-3) Assay procedures

Eight points were assayed within the antigen concentration range of 0 to 500 nM. 300 µL of the FRET CQ-body solution and 1.5 µL of the antigen with each concentration were added to each 1.5 mL Eppendorf tube and added in 3 portions (95 µL × 3) to a 96-well plate. In this operation, a 20 % human serum solution was also added as a blank to different wells in the same manner as above. The plates were left standing at room temperature for 20 minutes in the dark, followed by sample assay by the excitation of fluorescein and by the excitation of TAMRA. At the same time therewith, samples were prepared in the same manner as above using PBS instead of the 20% human serum and subjected to fluorescence intensity measurement.

### (3) Data processing

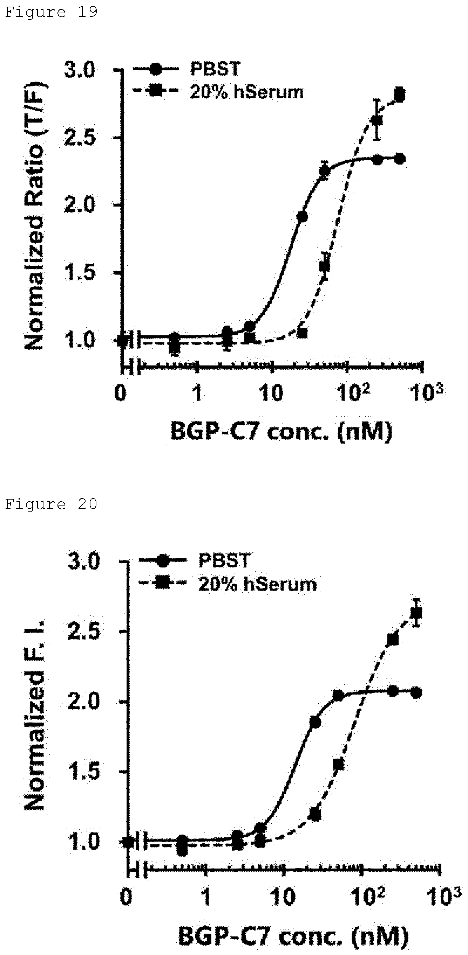

As for the excitation of fluorescein, the value of the blank was subtracted from all the measurements. The ratio between the fluorescence intensity of TAMRA and the fluorescence intensity of fluorescein (TAMRA/fluorescein) at each antigen concentration was taken for each measurement, and a mean and standard deviation were calculated (n = 3). Subsequently, the fluorescence intensity values at all the antigen concentrations were divided by the value of the fluorescence intensity ratio at the antigen concentration of 0 nM (normalization). Curve fitting was performed using a 4-parameter logistic curve as a regression model. Figure 19 shows a graph in which the abscissa of the obtained regression curve was expressed on a logarithmic scale. As for the excitation of TAMRA, the value of the blank was subtracted from all the measurements, and then, a mean and standard deviation were calculated (n = 3). Thereafter, the data was processed by the same procedures as in the excitation of fluorescein (Figure 20). As a result, fluorescence brightness was increased in a manner dependent on the concentration of BGP-C7, indicating that the antigen was able to be detected in real samples. Thus, the usefulness of FRET CQ-body in the field of clinical diagnosis, etc. was demonstrated.

### [Example 8] Measurement of BGP under conditions differing in probe concentration

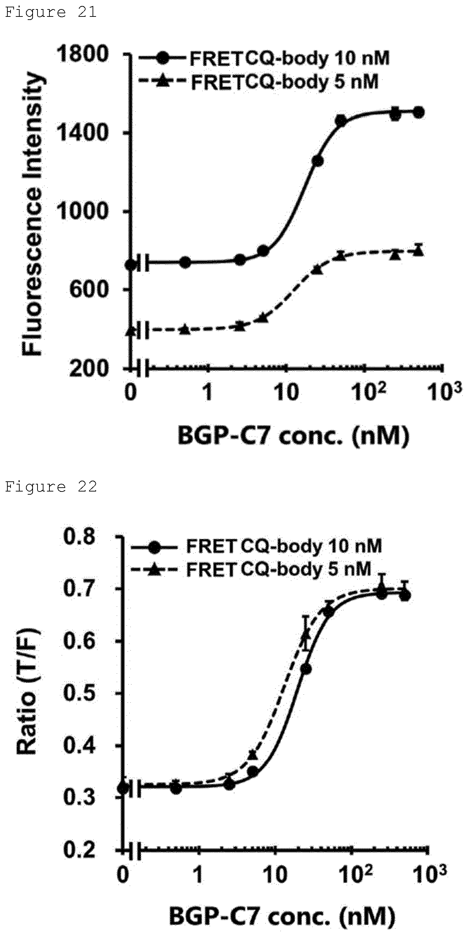

Since FRET CQ-body enables an antigen to be detected from change in fluorescence peak ratio, the antigen should be able to be accurately quantified even in a situation a probe concentration varies. In order to demonstrate this, fluorescence intensity was measured by the excitation of TAMRA and by the excitation of fluorescein in the presence of the antigen by the same procedures as in Example 6 in systems having a FRET CQ-body concentration of 5 nM or 10 nM. As a result, for the excitation of TAMRA, the fluorescence intensity at 10 nM FRET CQ-body was approximately two times the fluorescence intensity at 5 nM FRET CQ-body. Thus, the results were markedly influenced by the probe concentration (Figure 21). On the other hand, in the case of taking the fluorescence peak ratio between TAMRA and fluorescein by the excitation of fluorescein, calibration curves at 10 nM and 5 nM FRET CQ-body were consistent with each other, indicating that the antigen was able to be detected without being influenced by the probe concentration (Figure 22). Since the antigen can be accurately detected even in situations differing in probe concentration, the application of this technique to live cell imaging is also expected.

All publications, patents and patent applications cited herein are incorporated herein by reference in their entirety.

### [Industrial Applicability]

The present invention can be usefully used in, for example, the field of sample analysis or drug testing and the field of portable sample analysis kits

## Claims

1. A kit for antigen detection, comprising: a first peptide conjugated with two fluorescent dyes capable of forming a FRET pair or a fluorescent dye and a luminescent material capable of forming a BRET pair, and an antibody fragment with a second peptide added thereto, wherein 1) one of the first peptide and the second peptide is positively charged, and the other peptide is negatively charged, and 2) the first peptide and the second peptide are capable of forming a coiled coil.

2. The kit for antigen detection according to claim 1, wherein the first peptide comprises repeats of an amino acid sequence: X1-X2-X3-X4-X5-X6-X7 (wherein X1 and X6 each represent a positively charged amino acid, X7 represents a negatively charged amino acid, X2 and X5 each represent a hydrophobic amino acid, and X3 and X4 each represent any amino acid), and the second peptide comprises repeats of an amino acid sequence: X8-X9-X10-X11-X12-X13-X14 (wherein X8 and X13 each represent a negatively charged amino acid, X14 represents a positively charged amino acid, X9 and X12 each represent a hydrophobic amino acid, and X10 and X11 each represent any amino acid).

3. The kit for antigen detection according to claim 1, wherein the first peptide comprises repeats of an amino acid sequence: Lys Ile Ala Ala Leu Lys Glu, and the second peptide comprises repeats of an amino acid sequence: Glu Ile Ala Ala Leu Glu Lys.

4. The kit for antigen detection according to claim 1, wherein the first peptide consists of the amino acid sequence as set forth in SEQ ID NO: 1, and the second peptide consists of the amino acid sequence as set forth in SEQ ID NO: 2.

5. The kit for antigen detection according to any one of claims 1 to 4, wherein the two fluorescent dyes capable of forming a FRET pair are a rhodamine-based fluorescent dye and a fluorescein-based fluorescent dye.

6. The kit for antigen detection according to any one of claims 1 to 5, wherein the antibody fragment is Fab or camelid heavy chain antibody-derived VHH.

7. A method for detecting an antigen in a sample, comprising the following steps (1) and (2):
(1) contacting the sample with the kit according to any one of claims 1 to 6; and
(2) detecting the fluorescence of the fluorescent dye(s) conjugated with the first peptide.


SEQUENCE LISTING

<110> Tokyo Institute of Technology

<120> HOMOGENEOUS IMMUNOASSAY USING PEPTIDE CONJUGATED WITH MULTIPLE
FLUOROCHROMES

<130> FP-278PCT
<150> JP 2019/105006
<151> 2019-6-5

<160> 7

<170> PatentIn Ver. 2.1

<210> 1
<211> 29
<212> PRT
<213> Artificial Sequence
<220>
<223> Designed peptide to form coiled coils

<400> 1
Lys Ile Ala Ala Leu Lys Glu Lys Ile Ala Ala Leu Lys Glu Lys Ile
1               5                   10                  15
Ala Ala Leu Lys Glu Lys Ile Ala Ala Leu Lys Glu Cys
            20                  25

<210> 2
<211> 28
<212> PRT
<213> Artificial Sequence
<220>
<223> Designed peptide to form coiled coils

<400> 2
Glu Ile Ala Ala Leu Glu Lys Glu Ile Ala Ala Leu Glu Lys Glu Ile
1               5                   10                  15
Ala Ala Leu Glu Lys Glu Ile Ala Ala Leu Glu Lys
            20                  25

<210> 3
<211> 7
<212> PRT
<213> Artificial Sequence
<220>
<223> Designed peptide to form coiled coils

<400> 3
Lys Ile Ala Ala Leu Lys Glu
1               5

<210> 4
<211> 7
<212> PRT
<213> Artificial Sequence
<220>
<223> Designed peptide to form coiled coils

<400> 4

Glu Ile Ala Ala Leu Glu Lys
1               5

<210> 5
<211> 26
<212> DNA
<213> Artificial Sequence

<220>
<223> Description of Artificial Sequence:primer

<400> 5
tcagctagca tggctgaaat cgctgc                                          26

<210> 6
<211> 21
<212> DNA
<213> Artificial Sequence

<220>
<223> Description of Artificial Sequence:primer

<400> 6
atgctagtta ttgctcagcg g                                               21

<210> 7
<211> 24
<212> DNA
<213> Artificial Sequence

<220>
<223> Description of Artificial Sequence:primer

<400> 7
cgcggatccc tgaccggtgc ctcc                                            24